# FASCICULE DE BREVET EUROPEEN

(11) **EP 3 316 774 B1**
(45) Date de publication et mention de la délivrance du brevet: **09.12.2020**
(21) Numéro de dépôt: 16742368.0
(22) Date de dépôt: 01.07.2016
(51) Int. Cl.: G06Q 30/02, A61B 5/0484, A61B 5/00, A61B 5/16, G01N 33/18, A61B 5/02, A61B 5/053, A61B 5/0496

(54) **MÉTHODE DE CONSTRUCTION D'UN ESPACE SENSORIEL**
VERFAHREN ZUM AUFBAU EINES SINNESRAUMS
METHOD FOR CONSTRUCTING A SENSORY SPACE

(30) Priorité: 03.07.2015 FR 1556350
(43) Date de publication de la demande: 09.05.2018
(73) Titulaire: Centre Scientifique Et Technique Du Batiment (CSTB), 77420 Champs sur Marne (FR)
(72) Inventeur: DE OLIVEIRA, Fabrice, 44323 Nantes Cedex 3 (FR); HUMEAU, Philippe, 44323 Nantes Cedex 3 (FR); HAESE, Gwénaëlle, 44240 Sucé sur Erdre (FR); LE CLOIREC, Pierre, 35708 Rennes Cedex 07 (FR)
(74) Mandataire: Plasseraud IP
(86) Numéro de dépôt international: PCT/FR2016/051672
(87) Numéro de publication internationale: WO 2017/006032

(56) Documents cités:
- US-A1- 2009 327 068
- S. ROUSMANS ET AL: "Autonomic Nervous System Responses Associated with Primary Tastes", CHEMICAL SENSES, vol. 25, no. 6, 1 décembre 2000 (2000-12-01), pages 709-718, XP055262615, DOI: 10.1093/chemse/25.6.709
- GWENAELLE HAESE, PHILIPPE HUMEAU, FABRICE DE OLIVEIRA, PATRICK LE CALLET, PIERRE LE CLOIREC: "Prédiction de la perception sensorielle des goûts de l'eau par l'utilisation de mesures physiologiques : la microcirculation cutanée", L'ACTUALITÉ CHIMIQUE, vol. 390, 1 novembre 2014 (2014-11-01), pages 65-67, XP008179745,
- BREDIE WENDER L ET AL: "A Comparative Study on Facially Expressed Emotions in Response to Basic Tastes", CHEMOSENSORY PERCEPTION, SPRINGER NEW YORK LLC, US, vol. 7, no. 1, 31 janvier 2014 (2014-01-31), pages 1-9, XP035331783, ISSN: 1936-5802, DOI: 10.1007/S12078-014-9163-6 [extrait le 2014-01-31]
- GWENAELLE HAESE ET AL: "Objectivation of sensory measurements of off-flavours in water using central and autonomic nervous systems responses", 10TH IWA SYMPOSIUM ON OFF-FLAVOURS IN THE AQUATIC ENVIRONMENT, OCT 2013, TAINAN, TAIWAN; 2013-10-27 - 2013-11-01, INTERNATIONAL WATER ASSOCIATION (IWA), UK, 1 octobre 2013 (2013-10-01), pages 1-16, XP008179747,
- GWENAELLE HAESE ET AL: "Tastes and Odors of Water-Quantifying Objective Analyses: A Review", CRITICAL REVIEWS IN ENVIRONMENTAL SCIENCE AND TECHNOLOGY, vol. 44, no. 22, 17 novembre 2014 (2014-11-17), pages 2455-2501, XP055262617, ISSN: 1064-3389, DOI: 10.1080/10643389.2013.829972
- HASHIDA J C ET AL: "EEG pattern discrimination between salty and sweet taste using adaptive Gabor transform", NEUROCOMPUTING, ELSEVIER SCIENCE PUBLISHERS, AMSTERDAM, NL, vol. 68, 1 octobre 2005 (2005-10-01), pages 251-257, XP027809758, ISSN: 0925-2312 [extrait le 2005-10-01]

## Description

La présente invention concerne une méthode de construction d'un Espace Sensoriel basé sur des Réponses Physiologiques (ESRP) (à partir de paramètres physiologiques discriminants) ainsi qu'une méthode d'analyse sensorielle comprenant le positionnement d'un échantillon au sein d'un espace sensoriel (ESRP) de référence permettant la prédiction de ses caractéristiques chimiques et/ou sensorielles.

L'analyse sensorielle, aussi appelée métrologie sensorielle, est un domaine scientifique utilisé pour évaluer, mesurer, analyser et interpréter les réponses à des produits perçus par les sens de la vue, de l'odorat, du toucher, du goût et de l'audition. L'objectif principal de l'analyse sensorielle est de traduire les désirs et les préférences des consommateurs en propriétés bien définies et tangibles d'un produit donné. L'hypothèse utilisée pour les relier est qu'une partie des sensations est structurée par la préférence. En comparant et en analysant les caractéristiques du produit que les consommateurs apprécient ou non, l'analyse sensorielle contribue à identifier les aspects positifs et négatifs et à les adapter pour satisfaire les goûts des consommateurs.

Plusieurs aspects d'un produit sont intéressants à étudier : sa qualité, son intensité et sa dimension hédonique. La qualité est définie comme un ensemble de caractéristiques inhérentes telles que la description d'un visuel, d'une odeur, d'un toucher, d'un goût ou d'un son. L'intensité représente le degré de présence d'une caractéristique dans le produit. La dimension hédonique d'un produit est sa capacité à procurer du plaisir ou du déplaisir. Il existe trois principales catégories d'analyses sensorielles, visant à évaluer respectivement la qualité, l'intensité ou la dimension hédonique, et qui utilisent des panélistes choisis sur la base de critères spécifiques. Pour l'évaluation de la dimension hédonique, certaines méthodes d'analyses sensorielles peuvent faire appel à un panel naïf (non entraîné) mais nécessitent un nombre de panélistes important pour obtenir des résultats représentatifs. Au contraire, les méthodes classiques d'analyses sensorielles permettant l'évaluation de la qualité et de l'intensité font appel à des groupes de panelistes qu'il est généralement nécessaire de former et d'entraîner, notamment pour réduire la subjectivité de chacun des panelistes. La fiabilité des mesures, et particulièrement la reproductibilité, est alors fortement dépendante de la formation et de l'expertise des panélistes. La formation et l'entraînement des panélistes sont d'autant plus critiques dans certains domaines pour lesquels les caractéristiques à évaluer sont proches des seuils de détection. Par exemple, l'analyse sensorielle de l'eau potable reste extrêmement compliquée du fait des faibles concentrations des substances sapides et odorantes, et de la complexité à décrire un tel milieu. Dans tous les cas, la nécessité de formation et d'entraînement des panelistes et/ou d'un grand nombre de panelistes rend le recours aux analyses sensorielles coûteuses en temps et en argent. De plus, même lorsqu'elles sont réalisées par un panel entraîné, les analyses sensorielles donnent des résultats qui sont généralement interprétés de manière qualitative.

Par ailleurs, certaines méthodes d'analyses sensorielles proposent d'utiliser des indicateurs physiologiques. Des exemples d'analyses sensorielles utilisant des indicateurs physiologiques sont décrits dans Haese et al. Actualité chimique, vol 390, p.65-67, dans Wender et al. Chem. Percept., vol.7, p.1-9 et dans Haese et al. 10TH IWA Symposium on off-flavours in the Aquatic Envrionment. L'état émotionnel lié à des stimulations sensorielles s'exprime pour tout individu par des réactions comportementales et neuro-végétatives. Ces réactions ou effets physiologiques de l'activité du système nerveux autonome (SNA) peuvent être décelées plus ou moins directement par des indicateurs physiologiques (réponse électrodermale, fréquence cardiaque, indicateurs thermovasculaires, analyse du diamètre pupillaire, électroencéphalogramme...). Plusieurs études ont mis en évidence l'existence de corrélations entre des stimulations sensorielles et certains indicateurs physiologiques. Ces études ont notamment tenté de corréler les réponses physiologiques des individus à leurs préférences ou à différentes caractéristiques d'un stimulus. Les paramètres physiologiques extraits du suivi des indicateurs physiologiques varient selon la mesure (paramètres statistiques, paramètres de forme, paramètres spectraux, ...). Des études utilisant une approche multiparamétrique ont généralement extrait des paramètres statistiques de moyenne ou d'écart-type permettant de classer qualitativement les réactions physiologiques ou d'analyser directement les distributions par des tests statistiques de comparaison sur un panel de sujets. Cependant, ces méthodes ne permettent pas une discrimination significative de l'intensité et/ou de l'appréciation globale des stimuli.

De plus, ces études ont été essentiellement focalisées sur des concentrations largement supérieures aux seuils de détection (au moins 10 fois supérieur) et ont généralement comparé des stimuli de valences hédoniques diamétralement opposées.

Dans ce contexte, les inventeurs ont eu le mérite de mettre au point une méthode d'analyse sensorielle basée sur la construction d'un espace sensoriel individuel, c'est-à-dire spécifique à un individu, à partir de mesures physiologiques permettant de pallier les limites mentionnées ci-dessus. En particulier, la méthode d'analyse sensorielle selon l'invention est applicable à des sujets naïfs et permet ainsi de contourner le problème de l'entraînement long, contraignant et coûteux qu'impliquent les méthodes d'analyses sensorielles classiques. D'autre part, l'approche individuelle de cette méthode permet l'obtention d'un espace sensoriel de référence fiable et stable dans le temps. L'individu est assimilé à un capteur des caractéristiques sensorielles à étudier ce qui permet de s'affranchir des difficultés de verbalisation et des problèmes de subjectivité liées à la perception. Il s'agit de construire l'espace sensation qui au même titre qu'une fonction d'étalonnage, traduit la réponse physiologique individuelle mesurée à un stimulus en réponse sensorielle ou même en valeur caractéristiques du produit (chimique ou physique). Ceci est dû à la prédisposition particulière de l'organisme qui fait qu'un individu réagit d'une manière personnelle à l'influence d'agents extérieurs. La méthode s'appuie donc sur le principe de l'idiosyncrasie, c'est-à-dire sur l'exploitation de la prédisposition particulière d'un individu qui fait qu'un individu réagit d'une manière personnelle à l'influence d'agents extérieurs. Ainsi, il est possible d'obtenir des informations sur la qualité, l'intensité et/ou la dimension hédonique d'un échantillon inconnu souvent de façon plus fiable qu'avec une méthode d'analyse classique, notamment pour des stimuli proches des seuils de perception.

Ainsi, la présente invention porte sur une méthode de construction d'un espace sensoriel d'un individu ainsi que sur une méthode d'analyse sensorielle d'un échantillon basée sur l'utilisation de cet espace sensoriel.

La méthode de construction d'un espace sensoriel spécifique à un individu selon l'invention comprend:
- la réalisation d'une évaluation sensorielle d'au moins 4 stimuli de base représentatifs du sens à étudier par l'individu, les stimuli ayant une intensité (ou puissance ou niveau) inférieure à 3 fois l'intensité détectable par le sens à étudier;
- l'enregistrement des signaux relatifs à plusieurs indicateurs physiologiques au cours de l'évaluation sensorielle ayant réagi lors des stimuli;
- l'extraction de paramètres physiologiques à partir des signaux enregistrés desdits indicateurs physiologiques;
- l'identification des paramètres physiologiques discriminants les réponses physiologiques par traitement statistique;
- la construction d'un espace sensoriel à partir des paramètres physiologiques discriminants identifiés par traitement statistique.

La méthode de construction d'un espace sensoriel individuel est basée sur la confrontation des réponses physiologiques et des réponses sensorielles mesurées simultanément lors de la réalisation d'une évaluation sensorielle de stimuli de base représentatifs du sens à étudier par l'individu, les stimuli ayant une intensité (ou puissance ou niveau) inférieure ou au voisinage du seuil de détection. Les réponses sensorielles sont obtenues par auto-évaluation de l'individu à l'aide d'un questionnaire sensoriel. Les réponses physiologiques sont obtenues par l'enregistrement des signaux relatifs à au moins un indicateur physiologique.

La réalisation d'une évaluation sensorielle d'au moins 4 stimuli de base représentatifs du sens à étudier consiste ainsi, à déterminer au moins 4 stimuli associés au sens retenu et à une famille de composés du sens étudié, par exemple les saveurs pour le goût.

Ainsi, on peut citer pour le goût les stimuli qui correspondent aux saveurs de base, à savoir, acide, amère, salée, sucrée et umami, mais aussi les goûts secondaires : médicinal, végétal, rassis, métallique, terreux, moisi, chimique, épicé, fruité, floral, aromatique. Ces stimuli sont capables de ne stimuler que le sens du goût et de simuler des valences hédoniques positives et négatives.

Pour l'odorat, on pourra choisir les stimuli suivants comprenant les types d'odeurs suivants : septique, marécageux soufré, chimique (chlore), hydrocarbure, médical, végétal, fruité, floral, terreux, moisi, rance, phénolique,
La méthode peut aussi être déployée pour étudier d'autre sens tel que l'ouïe et le toucher.

Pour chacun des stimuli, plusieurs paramètres vont être étudiés : sa qualité, son intensité (ou puissance ou niveau) et sa dimension hédonique. La qualité est définie comme « qualitas », c'est-à-dire un ensemble de caractéristiques inhérentes. L'intensité (ou puissance ou niveau) représente le degré de présence d'une caractéristique dans le produit. L'intensité correspond à des stimuli du goût ou de l'odorat, la puissance à des stimuli du toucher et le niveau à des stimuli de l'ouïe. La dimension hédonique d'un produit est sa capacité à procurer du plaisir ou du déplaisir.

Ces paramètres sont déterminés par une analyse sensorielle classique à l'aide d'un questionnaire défini à cet effet. Chaque testeur est placé dans un environnement climatisé et neutre propice à la dégustation tel que défini par la norme ISO 8589 (2010). Les stimuli auxquels ils sont chacun soumis sont transmis dans un ordre aléatoire, un stimulus «blanc » est utilisé en début d'analyse. Le nombre de stimuli à chaque séance d'analyse sensorielle est limité afin d'éviter toute fatigue sensorielle. Par exemple, dans le cas de stimuli gustatifs, une séance de dégustation sera limitée à environ 10 échantillons à tester. Afin de déterminer également les seuils de détection des différents stimuli, pour un stimulus donné, différentes concentrations ou puissances ou niveaux sont testées. Dans la présente invention, on entend par « stimuli ayant une intensité (ou puissance ou niveau) inférieure ou au voisinage du seuil de détection du sens à étudier», des stimuli qui sont à une concentration (ou puissance ou niveau) inférieure à la concentration (ou puissance ou niveau) détectable par le sens à étudier et pouvant aller jusqu'à une concentration (ou puissance ou niveau) inférieure à 3, de préférence inférieure à 2 fois la concentration (ou puissance ou niveau) détectable par le sens à étudier.

En effet, une réponse physiologique à un stimulus peut être mise en évidence alors même que l'individu ne perçoit pas ce stimulus, c'est-à-dire que même au-dessous du seuil de détection du sens à étudier, une réponse physiologique sera mise en évidence et permettra la mise en œuvre de l'invention.

A titre d'exemple, les seuils de détection pour les quatre goûts primaires acide, sucré, salé et amer, sont donnés par les concentrations des composés représentatifs suivants:
- « acide » : acide citrique : seuil de détection : 2-2,60 mmol.L⁻¹ (0.384-0.5 g.L⁻¹),
- « amer » : caféine : seuil de détection : 0.26-1,8 mmol.L⁻¹ (0.05-0.35 g. L⁻¹),
- « salé » : NaCl : seuil de détection : 10 mmol.L⁻¹ (0.584 g. L⁻¹),
- « sucré » : saccharose : seuil de détection : 20 mmol.L⁻¹ (6.846 g. L⁻¹).

Les indicateurs physiologiques qui permettent d'établir la relation avec la qualité, l'intensité et la valence hédonique des stimuli sont des indicateurs du système nerveux central et/ou du système nerveux autonome mesurés de façon synchrone. Selon l'invention, plusieurs indicateurs physiologiques sont utilisés.

Des indicateurs physiologiques du système nerveux central pouvant être utilisés sont l'activité du système nerveux central qui peut être mesurée par électroencéphalographie (EEG) ou par spectroscopie proche infrarouge fonctionnelle.

Des indicateurs physiologiques du système nerveux autonome pouvant être utilisés sont la fréquence cardiaque, la réponse électrodermale (RED), la température cutanée, la microcirculation cutanée, le réflexe gusto-facial mesuré par électromyographie, la réponse pupillaire, la réponse respiratoire, la pression sanguine.

Pour chacun des indicateurs physiologiques retenus, différents paramètres ont été mesurés et ceux qui sont apparus comme les plus discriminants pour les réponses physiologiques associées aux stimuli et en lien avec l'intensité et/ou la valence hédonique et/ou les caractéristiques physiques ou chimiques (par exemple concentration de l'échantillon) des stimuli ont été retenus.

Selon un mode de réalisation de l'invention, lorsque plusieurs indicateurs physiologiques sont mesurés, les mesures sont effectuées de manière synchronisée, c'est-à-dire selon la même échelle de temps et à une même fréquence d'acquisition, par exemple 1 kHz. Pour chaque indicateur, différents paramètres sont mesurés.

A titre d'exemple, si l'indicateur physiologique retenu est la fréquence cardiaque (FC), elle sera mesurée à partir de l'électrocardiogramme (ECG) et suite à la détection temps RR (détection du pic R du complexe QRS de l'ECG, construction d'un tachogramme). La mesure de ECG peut être réalisée à l'aide d'un amplificateur différentiel 3-Lead ECG, ou équivalent, les électrodes étant positionnées selon les recommandations pour la mesure cardiaque et les différents paramètres (utilisation d'algorithmes développés sous Matlab®) suivants pourront être mesurés de manière synchronisée :
**Durée :** Durée totale de la réaction
**Durée au maximum :** Durée avant que le maximum de la FC soit atteint
**Maximum bpm :** Maximum atteint de la fréquence cardiaque en bpm
**Augmentation en bpm :** Augmentation en bpm de la FC par rapport aux 15 s précédant la
   Stimulation
**Augmentation en** % : Augmentation en % de la FC par rapport aux 15 s précédant la stimulation
**Ecart-type bande 0 Hz** - **0,15 Hz** : Racine carrée de la somme de l'énergie présente dans la bande de fréquence 0 - 0,15 Hz sur la durée totale de la réaction
**Ecart-type bande 0,15 Hz** - **0,4 Hz :** Racine carrée de la somme de l'énergie présente dans la bande de fréquence 0,15 - 0,4 Hz sur la durée totale de la réaction
**Ecart-type bande 0,4 Hz** - **0,5 Hz :** Racine carrée de la somme de l'énergie présente dans la bande de fréquence 0,4 - 0,5 Hz sur la durée totale de la réaction **(VLF+LF)/HF** : Ecart-type bande 0 Hz - 0,15 Hz/ Ecart-type bande 0,15 Hz - 0,4 Hz.

A titre d'exemple, si l'indicateur physiologique retenu est la RED, elle pourra être mesurée à l'aide d'un amplificateur GSR FE116 (Galvanic Skin Response) et les paramètres qui seront extraits pourront être :
- **APenteMax**: Ordonnée à PenteMax
- **APenteMin**: Ordonnée à PenteMin
- **APic**: Ordonnée au pic, non relative à la ligne de base
- **Hauteur**: Valeur de APic moins la ligne de base
- **PenteMax**: A chaque point d'échantillonnage, dans la zone entre le Début et la Fin, une pente est calculée par regression linéaire en cinq points centrée sur le point d'échantillonnage. Le maximum de ces pentes est pris pour PenteMax.
- **PenteMin**: Comme pour PenteMax, le minimum de ces pentes est pris pour PenteMin (où le minimum est la pente descendante la plus raide).
- **AirePic**: Aire de la zone entre le Début et la Fin
- **PenteDescente**: Pente de la ligne droite entret les points situés au croisement des 90% et 10% de la Hauteur du pic entre le pic et la Fin
- **PenteMontée**: Pente de la ligne droite entret les points situés au croisement des 10% et 90% de la Hauteur du pic entre le Début et le Pic
- **TDescente**: Durée entre les points situés au croisement des 90% et 10% de la Hauteur du pic entre le Début et le pic
- **DuréePic**: Durée entre le Début et APic
- **TPenteMax**: Durée entre le Début et PenteMax.
- **TPenteMin**: Durée entre le Début et PenteMin.
- **TMontée**: Durée entre les points situés au croisement des 10% et 90% de la Hauteur du pic entre le Début et le pic
- **Largeur30,**: LargeurX (où X égale soit 30, 50 ou 90) est calculée comme la
- **Largeur50,**: durée à X% de la hauteur du pic en partant de la ligne entre la
- **Largeur90**: montée et la descente
- **Largeur**: Durée entre le Début et la Fin

A titre d'exemple également, si l'indicateur physiologique est la microcirculation cutanée, elle pourra être mesurée à l'aide d'un appareil Periflux PF 5010 (Perimed AB, Sweden) et les paramètres mesurés simultanément qui seront extraits pourront être similaires aux paramètres extraits sur les signaux de RED.

L'identification des paramètres discriminants est faite par analyse statistique. Les différentes méthodes d'analyse statistique pouvant être utilisées sont notamment l'analyse de la variance (ANOVA) qui permet d'évaluer l'effet d'un ou plusieurs facteurs qualitatifs sur une variable quantitative pour laquelle les mesures ont été effectuées avec plusieurs répétitions (Pagès, J., 2010, Statistique générale pour utilisateurs. Méthodologie. PUR, Rennes) ; l'analyse des corrélations réalisée grâce au coefficient de corrélation de Pearson (ou de Spearman), qui est adaptée aux variables continues et est aussi le plus largement utilisé (Labbe, D., Rytz, A. & Hugi, A., 2004, Training is a critical step to obtain reliable product profiles in a real food industry context. Food Quality and Preference, 15, 341-348), pour la mesure de la performance du panel, ainsi que pour la mesure du lien entre les variables physiologiques et les variables sensorielles ; l'analyse en composantes principales (ACP) qui s'applique à des jeux de données croisant des individus en ligne et des variables quantitatives en colonne et dont l'objectif est de résumer la variabilité contenue dans l'ensemble des données par un minimum de dimensions non corrélées, appelées composantes principales (Escofier, B. & Pagès, J., 2008, Analyses factorielles simples et multiples : objectifs, méthodes et interprétation. Dunod, Paris) ; l'analyse factorielle des correspondances (AFC) qui s'applique à des tableaux de contingence croisant les modalités de deux variables qualitatives et dont l"objectif est d'obtenir une typologie des lignes et une typologie des colonnes, puis de les relier entre elles (Escofier, B. & Pagès, J., 2008, Analyses factorielles simples et multiples : objectifs, méthodes et interprétation. Dunod, Paris). D'autres méthodes d'analyse statistique multivariée ou de technique d'analyse de données exploratoire peuvent aussi être utilisées (Linear discriminant analysis : LDA, Partial least Square : PLS, réseaux de neurone...)

Une fois les paramètres discriminants déterminés pour chacun des individus séparément, un espace sensoriel « produits » individuel est construit.

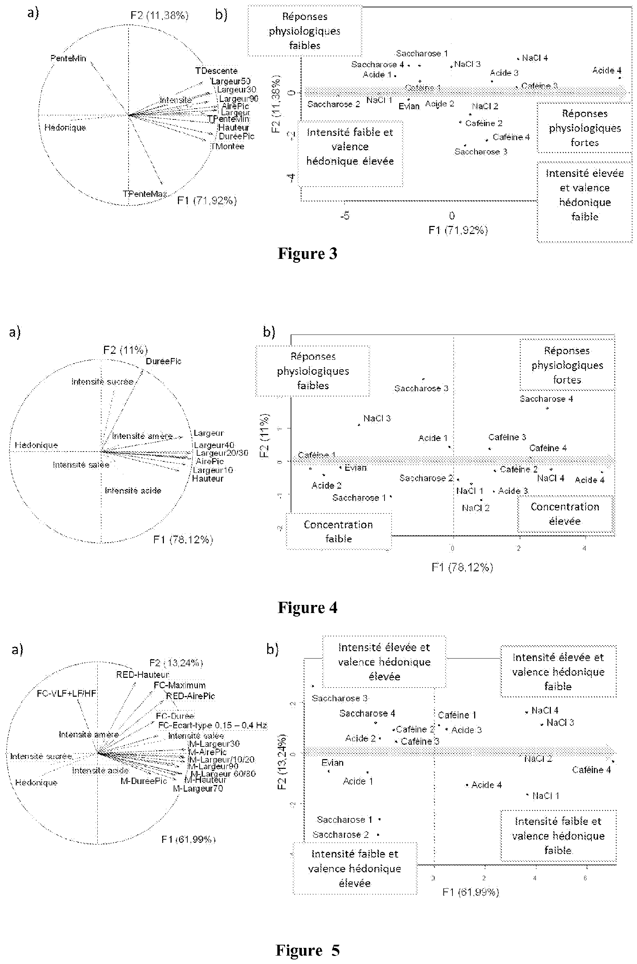

A titre d'exemple, un espace sensoriel est représenté sur la Figure 3b.

Cet espace sensoriel produits propre à l'individu, correspond à « l'étalonnage » de l'individu testeur et pourra ultérieurement être utilisé pour l'évaluation systématique d'échantillons par cet individu.

Ainsi, selon un autre aspect, l'invention porte également sur une méthode d'analyse sensorielle d'un échantillon comprenant :
- la construction d'un espace sensoriel d'un individu telle que définie précédemment;
- l'évaluation de l'échantillon par l'individu associée à l'enregistrement des signaux relatifs à au moins un indicateur physiologique correspondant aux paramètres physiologiques discriminants utilisés pour la construction de l'espace sensoriel ;
- l'extraction des paramètres physiologiques discriminants de l'échantillon ;
- le positionnement de l'échantillon dans l'espace sensoriel à partir des paramètres physiologiques discriminants de l'échantillon.

Grâce à cette méthode d'analyse sensorielle, des stimuli au voisinage des valeurs seuil de détection par les méthodes purement sensorielles classiques sont détectées. La sensibilité de la méthode est supérieure à celle des méthodes classiques et ce même avec des testeurs n'ayant pas subi d'entrainement spécifique mais présentant des paramètres physiologiques discriminants déterminés.

Ainsi, cette méthode peut permettre d'évaluer des échantillons inconnus pour mesurer à quelle place ils vont se situer par rapport à des stimuli connus, c'est-à-dire de déterminer leur qualité, intensité et valeur hédonique notamment.

L'invention porte également sur l'utilisation de la méthode d'analyse sensorielle (senso-physiologique) pour l'analyse de produits nécessitant des analyses sensorielles très pointues devant être réalisées par des testeurs de haute qualité qui suivent un entrainement préalable intense, comme par exemple l'eau potable.

L'invention va maintenant être décrite de façon plus détaillée à l'aide d'exemples donnés à titre d'illustration uniquement et des dessins annexés sur lesquels :
la figure 1 est un graphe de l'analyse de pic induit par un stimulus gustatif, réalisée par le logiciel Labchart®;
la figure 2 représente les boîtes de dispersion des notes d'intensité obtenues pour chaque stimulus gustatif et pour chaque concentration (1, 2, 3, 4, cf. Tableau 1), moyennées sur 4 sujets avec 6 répétitions chacun (i.e. un total de 24 notes d'intensité par produit), 12 répétitions pour l'échantillon d'Evian (i.e. un total de 48 notes d'intensité) ;
la Figure 3a) est la représentation des variables (actives en trait continu et illustratives en trait pointillé) ; et la figure 3b) est la représentation des stimuli gustatifs obtenue par APC (F1-F2) sur des données de microcirculation sanguine obtenues pour un individu.
la figure 4a) est la représentation des variables (actives en trait continu et illustratives en trait pointillé) ; et la figure 4b) est la représentation des stimuli gustatifs obtenue par APC (F1-F2) sur des données de réponse électrodermale obtenues pour un individu (sujet 1).
la figure 5a) est la représentation des variables (actives en trait continu et illustratives en trait pointillé) ; et la figure 5b) est la représentation des stimuli gustatifs obtenue par APC (F1-F2) obtenues pour un individu sur des données issus de plusieurs indicateurs physiologiques : microcirculation cutanée, réponse électrodermale et fréquence cardiaque.
La figure 6 présente un exemple d'utilisation de l'ESRP (construit à partir de la microcirculation) à partir de la projection de produit test ayant des concentrations de références,
La figure 7 présente un exemple d'utilisation de l'ESRP (construit à partir de la microcirculation) à partir de la projection de produit nouveaux et de modalité sensorielle différente (odeur Chlore),
La figure 8 illustre la qualité des modèles de prédiction pour la saveur salée issus de des paramètres de l'ESRP en comparant les concentrations réelle des échantillons de référence et leurs concentrations prédites (Q² = 0.995).

### EXEMPLES

**Exemple 1** : Construction d'un espace sensoriel destiné à être utilisé pour tester la qualité de l'eau du robinet sur un panel d'expert

L'objectif de cet exemple est de construire un espace sensoriel ESRP sur la base de mesures de la microcirculation cutanée afin de l'utiliser comme référence pour caractériser les problèmes d'odeur et de goût dans des échantillons d'eau. Les dimensions sensorielles étudiées sont la valence hédonique et l'intensité du produit.

### MATERIELS ET METHODES

### Sujets

Dans cet essai, quatre volontaires (2 hommes et 2 femmes) non-fumeurs ont été choisis parmi 12 personnes pour leur performance dans la reconnaissance des goûts. Leur moyenne d'âge était de 32 ans, de 24 à 40 ans. Ils ont attesté ne pas suivre de traitement médical et être exempts de troubles olfactifs et gustatifs et ont été informés des tests auxquels ils allaient être soumis. Ces quatre personnes ont été entrainées pendant plusieurs mois pour la reconnaissance et la détection des quatre goûts de base, ainsi que pour les procédures d'analyse sensorielle et physiologique.

### Stimuli de goût

Les quatre goûts de base (sucré, sale acide et amer) ont été utilisés. Les solutions ont été préparées avec de l'acide citrique pour le goût acide, la caféine pour le goût amer, le chlorure de sodium pour le goût salé et le saccharose pour le goût sucré (tous achetés chez Sigma Aldrich, France) dans de l'eau d'Evian. L'eau d'Evian a également été utilisée comme témoin et blanc. Chaque goût a été préparé avec les quatre concentrations mentionnées dans le Tableau 1.

**Tableau 1: Concentrations (mmol.L¹) pour chaque stimulus^{a} gustatif.**

| **Dilution** | **Acide citrique (acide)** | **Caféine (amer)** | **NaCl (salé)** | **Saccharose (sucré)** |
|---|---|---|---|---|
| 1 | 1.6 | 0.72 | 12 | 8 |
| 2 | 2.0 | 0.88 | 17 | 13 |
| 3 | 2.5 | 1.13 | 24 | 21 |
| 4 | 3.1 | 1.39 | 34 | 35 |

| | | | | |
|---|---|---|---|---|
| ^{a} Par exemple, dans ce tableau, la dilution 1 de l'acide citrique (correspondant à une concentration de 1,6 mmol.L⁻¹ d'acide citrique) sera dénommée Acide 1 dans cet exemple, la même dénomination sera utilisée pour les autres concentrations | | | | |

L'eau minérale d'Evian a été choisie pour son gout neutre (Teillet, E., Schlich, P., Urbano, C., Cordelle, S. & Guichard, E., 2010, Sensory methodologies and the taste of water. Food Quality and Preference, 21, 967-976.) et parce qu'elle a été largement utilisée dans des études de réactions physiologiques en réponse à des stimuli gustatifs (Rousmans, S., Robin, O., Dittmar, A. & Vernet-Maury, E., 2000 Autonomic nervous system responses associated with primary tastes. Chemical Senses, 25, 709-718.; Robin, O., Rousmans, S., Dittmar, A. & Vernet-Maury, E., 2003, Gender influence on emotional responses to primary tastes. Physiology & Behavior, 78, 385-393.; Leterme, A., Brun, L., Dittmar, A. & Robin, O. (2008) Autonomie nervous system responses to sweet taste: Evidence for habituation rather than pleasure. Physiology & Behavior, 93, 994-999.). La détection des concentrations seuils des goûts de base sont relativement élevés:
- environ 2 mmol.L⁻¹ pour l'acide citrique,
- environ 10 mmol.L⁻¹ pour NaCl
- environ 20 mmol.L⁻¹ pour le saccharose (Purves, D., Augustine, G.-J., Fitzpatrick, D. & Hall, W.-C., 2005, Neurosciences. De Boeck Supérieur, Bruxelles.) et
- entre 0,26 et 1,80 mmol.L⁻¹ pour la caféine (Robinson, K.M., Klein, B.P. & Lee, S.Y., 2005, Utilizing the R-index measure for threshold testing in model caffeine solutions. Food Quality and Preference, 16, 283-289.).

Les concentrations des solutions ont été choisies à partir de ces valeurs seuil et de tests de gouts antérieurs normés (AFNOR, 2012) afin d'avoir des concentrations proches des seuils de détection.

La quantité de solution à tester a été standardisée à 10 mL. Afin d'imprégner la totalité de la cavité buccale, les sujets gardaient la solution dans leur bouche pendant 5s avant de l'avaler.

### Système d'enregistrement de la microcirculation cutanée.

On a choisi un système Periflux PF 5010 (Perimed AB, Sweden) qui permet un suivi non invasif de perfusion sanguine dans les capillaires, artérioles et veinules.

L'unité de perfusion sanguine est arbitraire et correspond au produit de la vitesse moyenne de déplacement des hématies par leur concentration. Le système Periflux PF 5010 a un rayon laser de 780 nm, 1 mm de diamètre, sans effet thermique. Une sonde à fibre optique PR407 est utilisée. Le faisceau laser basse énergie est transmis par la sonde jusqu'au tissu. Une partie de la lumière se réfléchit sur les structures statiques et une autre partie de la lumière se réfléchit sur les cellules sanguines en mouvement. Lorsque la lumière se réfléchit à partir d'une cellule en mouvement, la longueur d'onde se modifie, ce qui correspond à l'effet Doppler. La lumière diffusée collectée par la fibre optique est utilisée pour calculer la valeur de la perfusion. La mesure est exprimée en unité de perfusion (UP). Les enregistrements ont été réalisés sur la pulpe de l'index de la main non dominante en raison de la forte vascularisation de cette zone. Les signaux de perfusion ont été enregistrés grâce au système d'acquisition à 8 canaux PowerLab PL35088/35, les données ont été collectées à une fréquence de 1 kHz et analysées par le logiciel Labchart® (AD Instruments Ltd, Royaume Uni).

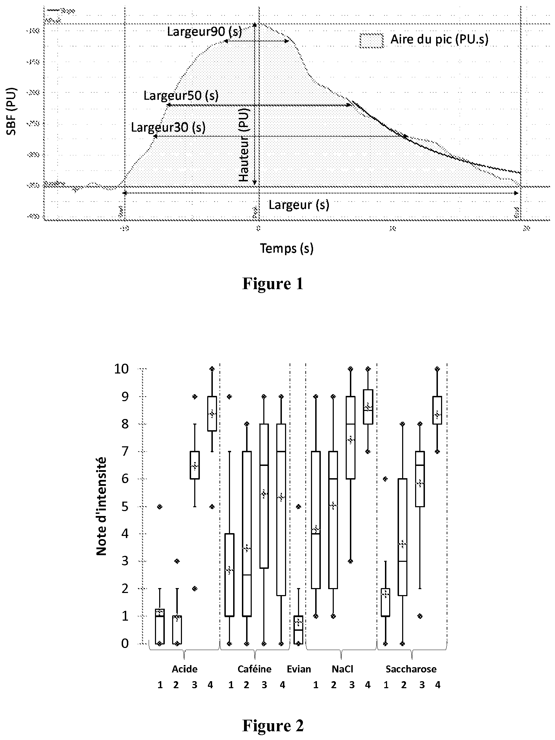

Le signal résultant a tout d'abord été inversé (multiplié par -1) de sorte que l'analyse du pic par le logiciel LabChart® soit possible, et sa valeur a été multipliée par 100 pour le convertir de volt en UP. Le signal a ensuite été filtré par un filtre passe-bas ayant une fréquence de coupure de 0,6 Hz pour retirer la fréquence caractéristique du battement cardiaque qui n'était pas pertinente dans la présente analyse. Ensuite, l'analyse du pic a été réalisée manuellement en choisissant le pic dans sa totalité et en l'analysant avec le logiciel LabChart® (Figure 1).

Plusieurs paramètres de forme ont été extraits du signal (i.e. amplitude, durée, pente). Ils sont donnés dans le Tableau 2.

**Tableau 2: Liste des paramètres extraits par le logiciel LabChart® et leur calcul**

| **Paramètres** | **Calcul** |
|---|---|
| **APenteMax** | Ordonnée à PenteMax |
| **APenteMin** | Ordonnée à PenteMin |
| **APic** | Ordonnée au pic, non relative à la ligne de base |
| **Hauteur** | Valeur de APic moins la ligne de base |
| **PenteMax** | A chaque point d'échantillonnage, dans la zone entre le Début et la Fin, une pente est calculée par régression linéaire en cinq points centrée sur le point d'échantillonnage. Le maximum de ces pentes est pris pour PenteMax. |
| **PenteMin** | Comme pour PenteMax, le minimum de ces pentes est pris pour PenteMin (où le minimum est la pente descendante la plus raide). |
| **AirePic** | Aire de la zone entre le Début et la Fin |
| **PenteDescente** | Pente de la ligne droite entre les points situés au croisement des 90% et 10% de la Hauteur du pic entre le pic et la Fin |
| **PenteMontée** | Pente de la ligne droite entre les points situés au croisement des 10% et 90% de la Hauteur du pic entre le Début et le Pic |
| **TDescente** | Durée entre les points situés au croisement des 90% et 10% de la Hauteur du pic entre le Début et le pic |
| **DuréePic** | Durée entre le Début et APic |
| **TPenteMax** | Durée entre le Début et PenteMax. |
| **TPenteMin** | Durée entre le Début et PenteMin. |
| **TMontée** | Durée entre les points situés au croisement des 10% et 90% de la Hauteur du pic entre le Début et le pic |
| **Largeur30,** | LargeurX (où X égale soit 30, 50 ou 90) est calculée comme la durée à X% |
| **Largeur50,** | de la hauteur du pic en partant de la ligne entre la montée et la descente |
| **Largeur90** | |
| **Largeur** | Durée entre le Début et la Fin |

### Procédure

Les sessions ont été individuelles et on a demandé aux sujets de ne pas manger ni boire pendant au moins 1 h avant le test. Ils sont arrivés 15 mn avant le début de la session afin de s'habituer aux conditions environnementales, en particulier la température de la pièce (maintenue constante à 23°C) et être au repos, assis sur une chaise confortable. Les 16 solutions pour tester et deux échantillons supplémentaires d'eau contenant uniquement de l'eau d'Evian, ajoutés aux références ont été présentés de manière monadique en suivant un ordre de test déterminé à partir d'un « balanced incomplete block design ». Pendant une session, seuls 9 échantillons sur 18 ont été présentés pour minimiser la fatigue sensorielle. En plus, un échantillon Evian a toujours été testé comme un échantillon « blanc » afin d'éviter l'effet de premier ordre (Lawless, H.T. & Heymann, H., 2010, Sensory evaluation of food: Principles and practices. Springer, New York) et a été exclu de l'analyse. Un signal lumineux a été envoyé pour donner instruction de tester l'échantillon. Lorsque la perturbation du système nerveux retrouvait son niveau de base (après environ 1 mn), un second signal lumineux indiquait aux sujets qu'ils devaient remplir un questionnaire concernant le stimulus précédemment testé. La première partie du questionnaire était liée à l'identification du goût (i.e. la détermination de la qualité du goût) ; les sujets devaient choisir entre cinq labels : sucré, salé, acide, amer ou neutre. Ensuite, ils devaient attribuer un score d'intensité du goût sur une échelle de 11 (de 0 « pas intense » (i.e. goût de l'Evian seulement) à 10 « très intense ») et finalement pour donner une note hédonique sur une échelle de 11 (de 0 « très déplaisant » à 10 « très plaisant », 5 étant « neutre »).

Pendant cette seconde phase, les sujets pouvaient rincer leur bouche avec de l'eau d'Evian et attendaient le signal lumineux suivant qui indiquait qu'ils pouvaient tester le stimulus suivant. Cette procédure a été répétée pour chacun des 9 stimuli. Une session a duré environ 45 mn incluant un débriefing à la fin du test pour s'assurer de comprendre le sens des variations de signal. L'expérience a été répétée 12 fois par sujet, ce qui signifie que les données soient faites de 6 données par sujet et par produit et 12 données pour le produit Evian par sujet. Et ainsi, la base de données consiste en 432 échantillons.

### Analyse Statistique

Toutes les analyses ont été réalisées avec un logiciel SensoMineR (Husson, F. & Lê, S. (2009) SensoMineR: Sensory data analysis with R. R package version 1.10. http://CRAN.R-project.org/package=SensoMineR) et FactoMineR (Lê, S., Josse, J. & Husson, F. (2008) FactoMineR: An R Package for Multivariate Analysis. Journal of Statistical Software, 25, 1-18.) réalisées en R (version 2.14.1) et XLSTAT-Pro 2010.3.01.

### Réponses sensorielles

L'effet du goût sur la note hédonique et d'intensité a été analysé par une analyse de variance (ANOVA) incluant l'effet produit, l'effet sujet et l'interaction produit-sujet. L'effet sujet a été fixé parce qu'il n'y avait que 4 sujets et que les résultats pouvaient seulement être appliqués à un schéma particulier. Les différences ont été considérées comme significatives à un niveau de 0,05.

### Variations de la microcirculation cutanée

L'Analyse du Composant Principal (ACP) a été utilisée:
- Pour étudier les individus (i.e. les stimuli de goût): deux stimuli sont proches s'ils partagent des résultats similaires, l'intérêt étant la variabilité entre les individus,
- Pour étudier les variables (i.e. les paramètres physiologiques): il permet la visualisation des corrélations entre les variables physiologiques afin de trouver des variables synthétiques,
- Pour lier les deux études par caractérisation des groupes d'individus avec des variables (Escofier, B. & Pagès, J., 2008, Analyses factorielles simples et multiples : objectifs, méthodes et interprétation. Dunod, Paris).

Les réponses SKBF ont été analysées de façon individuelle par ANOVA une voie (effet produit), les sujets ayant leur propre "canal préférentiel" (Lacey, J.I., Bateman, D.E. & VanLEHN, R., 1953, Autonomic response specificity: An experimental study. Psychosomatic Medicine, 15, 8-21.). En effet, certains sujets répondaient aux variations SKBF, d'autres avec des variations EDR, et des mesures physiologiques sont connues pour montrer d'importantes différences individuelles, qui conduisent à la nécessité d'individualiser l'analyse (Johannes, B. & Gaillard, A.W.K., 2014, A methodology to compensate for individual differences in psychophysiological assessment. Biological psychology, 96, 77-85).

Enfin, des corrélations entre les notes moyennes hédoniques et d'intensité par produit et les réponses moyennes du système nerveux ont été calculées à l'aide du coefficient de Pearson. Les différences ont été considérées comme significatives à un niveau de 0.05.

### RESULTATS

### Evaluation Sensorielle

### Taux de Reconnaissance

Les goûts salés et sucrés ont été bien identifiés par le panel pour toutes les concentrations avec un taux de reconnaissance de 100% pour les 3 notes les plus élevées (Tableau 3). Les concentrations acide les plus élevées ont également été parfaitement reconnues et le goût amer a été relativement bien identifié (respectivement 92% et 96% de réponses justes pour les concentrations 3 et 4). Cependant, des concentrations acides faibles n'ont pas été reconnues et ont été classées « neutre » la plupart du temps (respectivement 54% et 38% pour les concentrations 1 et 2). L'eau minérale d'Evian a souvent été perçue comme amère (dans 38% des cas), mais comme elle a été utilisée pour toutes les dilutions, les résultats sont comparables.

**Tableau 3: Notes de reconnaissance Individuelle pour chaque stimulus gustatif et les taux de reconnaissance (%) pour les quatre sujets**

| | | Acide citrique | | | | Caffeine | | | | Evian | Nacl | | | | Saccharose | | | |
|---|---|---|---|---|---|---|---|---|---|---|---|---|---|---|---|---|---|---|
| | | 1 | 2 | 3 | 4 | 1 | 2 | 3 | 4 | | 1 | 2 | 3 | 4 | 1 | 2 | 3 | 4 |
| Sujet 1 | Acide | 1 | 1 | 6 | 6 | 0 | 0 | 0 | 0 | 1 | 0 | 0 | 0 | 0 | 0 | 0 | 0 | 0 |
| | Amer | 1 | 1 | 0 | 0 | 5 | 6 | 6 | 6 | 4 | 0 | 0 | 0 | 0 | 0 | 0 | 0 | 0 |
| | Neutre | 3 | 4 | 0 | 0 | 1 | 0 | 0 | 0 | 2 | 0 | 0 | 0 | 0 | 0 | 0 | 0 | 0 |
| | Salé | 0 | 0 | 0 | 0 | 0 | 0 | 0 | 0 | 1 | 6 | 6 | 6 | 6 | 0 | 0 | 0 | 0 |
| | Sucré | 1 | 0 | 0 | 0 | 0 | 0 | 0 | 0 | 4 | 0 | 0 | 0 | 0 | 6 | 6 | 6 | 6 |
| Sujet 2 | Acide | 0 | 2 | 6 | 6 | 0 | 0 | 0 | 0 | 0 | 0 | 0 | 0 | 0 | 0 | 0 | 0 | 0 |
| | Amer | 0 | 0 | 0 | 0 | 6 | 4 | 6 | 6 | 3 | 0 | 0 | 0 | 0 | 0 | 0 | 0 | 0 |
| | Neutre | 5 | 3 | 0 | 0 | 0 | 1 | 0 | 0 | 7 | 0 | 0 | 0 | 0 | 0 | 0 | 0 | 0 |
| | Salé | 0 | 0 | 0 | 0 | 0 | 0 | 0 | 0 | 0 | 6 | 6 | 6 | 6 | 0 | 0 | 0 | 0 |
| | Sucré | 1 | 1 | 0 | 0 | 0 | 1 | 0 | 0 | 2 | 0 | 0 | 0 | 0 | 6 | 6 | 6 | 6 |
| Sujet 3 | Acide | 1 | 1 | 6 | 6 | 0 | 1 | 0 | 0 | 1 | 0 | 0 | 0 | 0 | 0 | 0 | 0 | 0 |
| | Amer | 2 | 4 | 0 | 0 | 6 | 5 | 6 | 6 | 3 | 0 | 0 | 0 | 0 | 0 | 0 | 0 | 0 |
| | Neutre | 3 | 1 | 0 | 0 | 0 | 0 | 0 | 0 | 8 | 0 | 0 | 0 | 0 | 3 | 0 | 0 | 0 |
| | Salé | 0 | 0 | 0 | 0 | 0 | 0 | 0 | 0 | 0 | 6 | 6 | 6 | 6 | 0 | 0 | 0 | 0 |
| | Sucré | 0 | 0 | 0 | 0 | 0 | 0 | 0 | 0 | 0 | 0 | 0 | 0 | 0 | 3 | 6 | 6 | 6 |
| Sujet 4 | Acide | 1 | 4 | 6 | 6 | 0 | 0 | 0 | 1 | 0 | 0 | 0 | 0 | 0 | 0 | 0 | 0 | 0 |
| | Amer | 3 | 1 | 0 | 0 | 3 | 5 | 4 | 5 | 8 | 1 | 0 | 0 | 0 | 0 | 0 | 0 | 0 |
| | Neutre | 2 | 1 | 0 | 0 | 3 | 1 | 2 | 0 | 4 | 0 | 0 | 0 | 0 | 0 | 0 | 0 | 0 |
| | Salé | 0 | 0 | 0 | 0 | 0 | 0 | 0 | 0 | 0 | 5 | 6 | 6 | 6 | 0 | 0 | 0 | 0 |
| | Sucré | 0 | 0 | 0 | 0 | 0 | 0 | 0 | 0 | 0 | 0 | 0 | 0 | 0 | 6 | 6 | 6 | 6 |
| Taux de reconnaissance % | Acide | 13 | 33 | 100 | 100 | 0 | 4 | 0 | 4 | 4 | 0 | 0 | 0 | 0 | 0 | 0 | 0 | 0 |
| | Amer | 25 | 25 | 0 | 0 | 83 | 84 | 92 | 96 | 38 | 4 | 0 | 0 | 0 | 0 | 0 | 0 | 0 |
| | Neutre | 54 | 38 | 0 | 0 | 17 | 8 | 8 | 0 | 44 | 0 | 0 | 0 | 0 | 12 | 0 | 0 | 0 |
| | Salé | 0 | 0 | 0 | 0 | 0 | 0 | 0 | 0 | 2 | 96 | 100 | 100 | 100 | 0 | 0 | 0 | 0 |
| | Sucré | 8 | 4 | 0 | 0 | 0 | 4 | 0 | 0 | 12 | 0 | 0 | 0 | 0 | 88 | 100 | 100 | 100 |

### Notes d'intensité

Les notes d'intensité étaient fortement liées au taux de reconnaissance (r = 0.75, *p* < 0.001). Ils ont confirmé que les faibles concentrations en acide 1 et 2 ont été notées comme peut intenses comme l'eau minérale d'Evian (respectivement 1.2 ± 1.5, 1.0 ± 1.0 et 0.8 ± 1.2); Elles étaient juste en dessous des seuils de détection. L'effet du produit était très significatif sur les notes d'intensité moyenne (p < 0.001). Les concentrations 4 des goûts acide, salé et sucré avaient les notes les plus élevées, respectivement 8.4 ± 1.2, 8.6 ± 1.0 et 8.3 ± 0.9. Les solutions amères étaient moins différentiées les unes des autres et ont donné les écart-types les plus élevés ; les concentrations 3 et 4 n'étaient pas significativement différentes. Les concentrations 1 et 2 du goût salé n'étaient pas significativement différentes non plus. Le seul goût pour lequel chaque concentration était notée de façon significativement différente de la précédente était le sucré (Figure 2).

L'interaction produit-sujet était significative (*p* < 0.001); l'effet du produit n'était pas le même en fonction du sujet. Cette interaction était principalement due au sujet ayant eu des difficultés à discriminer les goûts amers. L'entrainement tend à réduire cet effet dans le contexte d'une description des caractéristiques des produits (Lawless and Heymann, 2010).

### Notes hédoniques

Les notes hédoniques étaient significativement différentes entre les produits (*p* < 0.001). L'eau minérale d'Evian a obtenu une note hédonique neutre (5.2 ± 1.5), ainsi que les concentrations les plus faibles des différents goûts proches des seuils de détection. Au fur et à mesure que la concentration augmentait, les notes hédoniques des solutions acides, amères et salées diminuaient alors que les notes hédoniques des solutions sucrées augmentaient comme leurs concentrations.

La concentration 4 acide, les concentrations 3 et 4 de caféine et chacune des concentrations de NaCl avaient des notes hédoniques significativement inférieures aux autres solutions (respectivement 3.3 ± 2.4, 3.5 ± 1.8, 3.5 ± 2.3 et 1.3 ± 0.9), alors que toutes les solutions sucrées ont obtenu des notes hédoniques significativement plus élevées (7.0 ± 2.6 pour la concentration en saccharose la plus élevée).

L'interaction produit-sujet était significative (*p* < 0.001); l'effet du produit n'était pas le même en fonction du sujet. Dans le cas de notes hédoniques, cette interaction n'était pas problématique puisque plusieurs sujets pouvaient par exemple apprécier le goût acide alors que d'autres sujets le trouvaient très déplaisant.

Ces résultats sensoriels ont confirmé le choix des goûts de base et le choix des différentes concentrations. En effet, les 17 stimuli étaient répartis sur l'échelle d'intensité, des seuils de détection aux intensités les plus élevées, et ont permis l'expérience sur l'ensemble de l'échelle hédonique, même si la valence hédonique est prise comme une information individuelle dans cet exemple.

L'évaluation physiologique est focalisée sur les résultats du sujet 3 uniquement.

### Evaluation physiologique, ESRP : microcirculation sanguine cutanée

ACP a été réalisée afin de visualiser les données avec les paramètres physiologiques extraits des variations individuelles de SKBF à partir du sujet 3 (cf. Figure 1 et Tableau 2). Cette analyse permet la représentation de toutes les variables sur un cercle de corrélations (Figure 3a) et tous les produits sur un espace bi-dimensionnel (Figure 3b). La première carte représentait 83,3% de la variabilité initiale.

Le premier axe d'ACP était caractérisé par un groupe de variables de durée et d'amplitude de réponse physiologique (Figure 4a). De cette manière, les produits Acide 3 et 4, Caféine 3 et 4, NaCl 4, situés sur la partie droite de la carte (Figure 4b) avaient tendance à avoir des valeurs élevées pour les variables "largeur", "hauteur" et "aire de pic".

Le second axe séparait les variables de « pente ». Les produits situés dans la partie haute du graphe avaient tendance à provoquer une chute plus rapide du signal après la stimulation, alors que des produits dans la partie basse induisaient un retour plus lent au niveau de base avant stimulation.

Les variables hédoniques et d'intensité ont été traitées comme des variables d'illustration; elles n'ont pas participé à la construction des axes. Cependant, elles semblent être fortement corrélées aux variables physiologiques. L'intensité perçue était de façon importante et positive corrélée à la première dimension (r = 0.72, *p* = 0.001), alors que la variable hédonique était corrélée de façon importante et négative à la première dimension (r = -0.65, *p* = 0.005). Les produits perçus comme intenses et déplaisants semblaient être caractérisés par des réactions physiologiques plus fortes contrairement aux stimuli plaisants et intenses.

Selon l'ANOVA, les variables les plus discriminantes pour les produits étaient la largeur du signal, i.e. sa durée (*p* < 0.001), et l'aire du pic (*p* < 0.001). L'acide 4, NaCl 4 et Caféine 3 induisaient une perturbation significativement plus forte que les autres. Les paramètres étant mathématiquement liés, la hauteur du pic, i.e. son amplitude, était également un paramètre discriminant (*p* = 0.004) pour les stimuli Acide 4 et Caféine 3. L'analyse co-relationnelle a confirmé ces résultats. La largeur du signal était significativement corrélée à l'intensité (r = 0.68, *p* = 0.003) et au caractère plaisant (r = - 0.63, *p* = 0.007), ainsi que l'aire de pic (r = 0.65, *p* = 0.004 and r = -0.65, *p* = 0.005 respectivement).

### Exemple 2: Construction d'un espace sensoriel destiné à être utilisé pour tester la qualité de l'eau du robinet sur un panel naïf

L'objectif de cet exemple est de construire un espace sensoriel ESRP sur la base de mesures de la microcirculation cutanée, la réponse électrodermale et la fréquence cardiaque afin de l'utiliser comme référence pour caractériser les problèmes d'odeur et de goût dans des échantillons d'eau. Les dimensions sensorielles étudiées sont la valence hédonique et les intensités du produit en fonction des 4 saveurs de base (échelles d'intensité sucrée, d'intensité salée, d'intensité amère et d'intensité acide).

### Sujets

La sélection des panélistes pour le choix des sujets participant à la phase finale des expérimentations a été réalisée de la même manière que celle présentée lors de la phase d'expérimentations préliminaires. Les critères d'inclusion sont restés les mêmes. Les volontaires ont été recrutés selon les critères suivants :
- Avoir entre 18 et 45 ans,
- Etre non-fumeur,
- Ne pas suivre de traitement médical particulier autre que la contraception orale,
- Ne pas avoir de problèmes de goût ou d'odorat.

Trente et un sujets, dix-neuf femmes et douze hommes (âgés de 18 à 40 ans), ont participé aux épreuves de sélection réparties sur deux séances, après avoir été informés des conditions expérimentales et avoir signé un formulaire d'acceptation.

Les consignes à respecter afin de minimiser l'impact de paramètres indésirables sur la sensibilité aux goûts de l'eau ont été les suivantes :
- Ne pas boire de café ou de thé durant l'heure précédant la dégustation,
- Eviter l'emploi de parfum, d'after-shave ou de rouge à lèvre le jour de la dégustation,
- Ne pas manger de bonbon, chewing-gum ou pastille pour la gorge avant la dégustation,
- Ne pas manger épicé et ne pas boire d'alcool avant la dégustation,
- Espacer au maximum le moment du brossage de dent du moment de la dégustation.

### Stimuli de goût

Les quatre goûts de base (sucré, sale acide et amer) ont été utilisés. Les solutions ont été préparées avec de l'acide citrique pour le goût acide, la caféine pour le goût amer, le chlorure de sodium pour le goût salé et le saccharose pour le goût sucré (tous achetés chez Sigma Aldrich, France) dans de l'eau d'Evian. L'eau d'Evian a également été utilisée comme témoin et blanc. Chaque goût a été préparé avec les quatre concentrations mentionnées dans le Tableau 4.

**Tableau 4 : Concentrations utilisées pour chaque saveur en mmol.L⁻¹ (en g.L⁻¹)**

| **Dilution** | **Acide (acide citrique)** | **Amère (caféine)** | **Salée (NaCl)** | **Sucrée (saccharose)** |
|---|---|---|---|---|
| 1 | 1,98 (0,38) | 0,72 (0,14) | 8,21 (0,48) | 7,57 (2,59) |
| 2 | 2,13 (0,41) | 0,88 (0,17) | 11,81 (0,69) | 12,62 (4,32) |
| 3 | 2,50 (0,48) | 1,13 (0,22) | 16,77 (0,98) | 21,03 (7,20) |
| 4 | 3,12 (0,6)0 | 1,39 (0,27) | 23;96 (1,40) | 35,06 (12,00) |

### Evaluation physiologique, ESPR : réponse électrodermale,

L'ESPR est présenté sur les figures 4 a) et b) construit à partir de paramètres pertinents extraits de la réponse électrodermale. Ces figures présentent respectivement le cercle de corrélation du plan factoriel F1-F2 et le plan factoriel F1-F2 avec le nuage des individus. Toutes les variables sont positivement corrélées au premier axe, la variable « Durée Pic » est aussi positivement corrélée au deuxième axe. Sur le nuage des individus, tous les produits à la concentration 4 la plus élevée sont situés à droite du plan (contributions cumulées : 39,63%). Ces produits prennent des valeurs plus élevées pour l'ensemble des paramètres physiologiques. A l'inverse, les produits aux concentrations faibles Caféine 1, Acide 2 et l'échantillon d'eau d'Evian se trouvent à gauche du plan (contributions cumulées : 43,48%) et ont des valeurs plus faibles pour l'ensemble des paramètres. Le premier facteur oppose donc les faibles intensités aux fortes intensités. L'axe 2 est porté par les produits Saccharose 3 et 4 qui contribuent à plus de 58% de son inertie. Ces deux produits prennent des valeurs plus élevées pour la variable « Durée Pic ».

Comme montré dans l'étude préliminaire, le lien entre les variables sensorielles d'autoévaluation et les réponses physiologiques peut être visualisé grâce à la projection de ces premières en variables illustratives sur l'ACP. La corrélation est ensuite calculée entre les moyennes obtenues par produit.

Le cercle des corrélations et le premier plan factoriel sont représentés sur la figure 4.

Le premier facteur de l'ACP (F1) est aussi négativement corrélé aux notes hédoniques (r = -0,506, p < 0,05). En examinant les notes hédoniques données par le sujet 1, cette corrélation est expliquée par le fait que les notes hédoniques des produits baissent relativement à leur concentration qui augmente. Toutefois, les niveaux des notes hédoniques ne sont pas les mêmes entre les stimuli sucrés et les stimuli amers par exemple. En effet, le sujet 1 apprécie relativement les stimuli sucré, bien que la note soit plus basse pour le stimulus fort que le faible, tandis qu'il apprécie peu les stimuli amers. La RED chez le sujet 1 est donc plus liée à l'intensité des stimuli qu'à leur dimension hédonique.

Le lien entre les réactions physiologiques et la concentration réelle des goûts dans l'eau a été étudié grâce aux coefficients de corrélations. Les corrélations ont été calculées par goût.

La représentation de l'espace ESRP obtenue, figure 4, est directement liée aux concentrations. En effet, le premier facteur de l'ACP est significativement positivement corrélé à la concentration en acide citrique et à la concentration en saccharose (r = 0,829 p < 0,20 et r = 0,878 p < 0,20 respectivement) et est positivement corrélé à la concentration en NaCl et en caféine sans atteindre la significativité (r = 0,301 et r = 0,792, respectivement). Le deuxième facteur est lui aussi positivement corrélé à la concentration en caféine et à la concentration en saccharose (r = 0,690 et r = 0,756, respectivement). L'étude des corrélations entre chaque paramètre physiologique discriminant et la concentration montre un lien positif significatif entre la hauteur et l'aire du pic et la concentration en acide citrique (r = 0,895, p < 0,20 et r = 0,908, p < 0,10 respectivement). Concernant les concentrations en caféine et en NaCl, elles sont corrélées positivement et de manière significative aux temps de retour à un niveau physiologique de base. La concentration en saccharose est corrélée positivement à la durée au pic et à la durée de la perturbation (r = 0,937, p < 0,10 et r = 0,955, p < 0,05 respectivement).

### Evaluation physiologique, ESPR : microcirculation sanguine cutanée, réponse électrodermale, fréquence cardiaque

Cet exemple porte un espace sensoriel sur un individu ayant répondu sur plusieurs canaux. Cet exemple est présenté sur la figure 5 a) et b). Les résultats obtenus en RED et en variations de la fréquence cardiaque pour cet individu sont maintenant présentés. Seuls deux paramètres en RED sont discriminants pour les produits : l'aire (p < 0,20) et la hauteur du pic (p < 0,01) et quatre en VFC : le maximum en bpm (p < 0,10), la durée et l'écart-type de la bande 0,15 - 0,4 H (p < 0,20), et le VLF+LF/HF (p < 0,05). Ces six variables ont été ajoutées à l'ACP précédente avec les variables discriminantes de microcirculation.

L'aire du pic de RED est significativement corrélée à la valence hédonique des stimuli (r = -0,365, p < 0,20), ainsi que la durée, le maximum et l'écart-type de la bande 0,15 - 0,4 Hz des variations de la fréquence cardiaque (respectivement r = -0,819, p < 0,0001, r = -0,601, p < 0,05 et r = -0,659, p < 0,01). Le maximum atteint en fréquence cardiaque est lié positivement et de manière significative aux intensités salées et amères déclarées et la durée et l'écart-type de la bande 0,15 - 0,4 Hz sont aussi corrélées positivement à l'intensité salée. Ce sont donc les stimuli salés qui ont eu le plus d'impact sur les variations cardiaques. Les deux variables de RED sont directement liées à la concentration en NaCl et en saccharose : l'aire du pic est corrélée significativement et positivement (respectivement r = 0,935, p < 0,10 et r = 0,860, p < 0,20), ainsi que la hauteur (respectivement r = 0,971, p < 0,05 et r = 0,854, p < 0,20).

### Exemple 3 : Utilisation des ESRP avec des produits de références

L'ESRP peut être utilisé comme espace de réponse sur laquelle des produits peuvent être projetés afin d'être comparés à des produit étalons (produits étant à l'origine de la construction de l'espace sensoriel). Cette projection est réalisée à partir de l'analyse des paramètres physiologiques (sélectionnés pour la construction de l'ESRP) liés à la réponse physiologique de ces nouveaux produits.

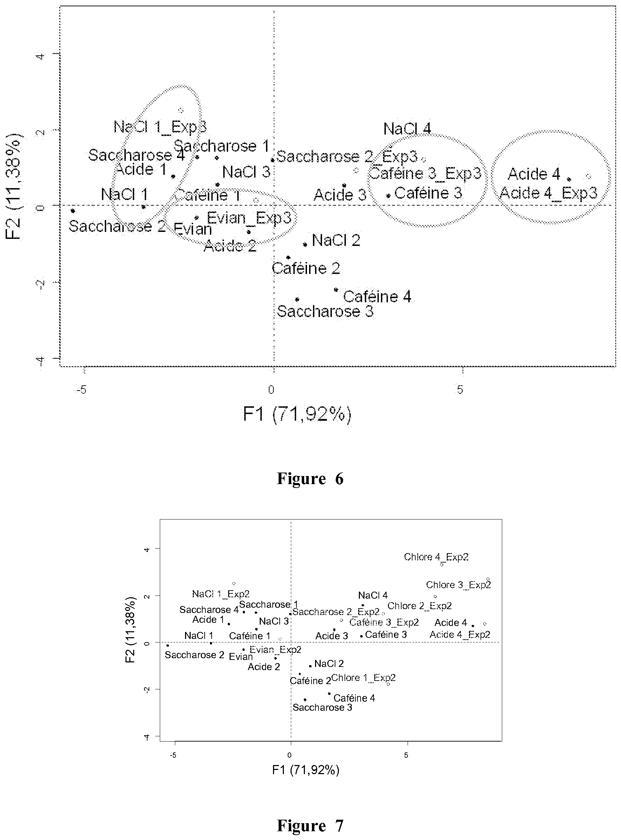

La figure 6 illustre la projection des nouvelles mesures ayant des concentrations de références. La projection de ces nouvelles mesures, en tant qu'individus illustratifs (indexé _Exp3), montre que la position des différents échantillons reste relativement inchangée sur le premier facteur et que le classement des échantillons est le même, à l'exception du Saccharose 2. En effet, les coordonnées de l'échantillon d'eau d'Evian, du NaCl 1, de la Caféine 3 et de l'Acide 4 pour la première expérimentation sont respectivement de -2,0, - 3,4, 3,0 et 7,8 sur le premier facteur de l'ACP et sont respectivement de -0,5, -2,4, 3,9 et 8,3 lors de la deuxième expérimentation. Ces résultats montrent que, même un an après, les mesures sont stables et reproductibles dans le temps. La représentation de l'ESPR individuel est donc robuste et peut effectivement être réutilisée pour des mesures postérieures. Il reste maintenant à étudier s'il est possible de l'utiliser pour de nouveaux échantillons à évaluer avec d'autres goûts ou odeurs que ceux utilisés pour sa construction.

### Exemple 4 : Utilisation des ESRP avec des nouveaux produits (odeur de chlore)

L'odeur de chlore semble pertinente à examiner pour plusieurs raisons. En effet, une des odeurs les plus problématiques dans l'eau potable en France est celle du chlore. Le chlore est davantage une odeur qu'un goût puisque sa détection dans l'eau se fait sur la base d'une composante olfactive et non gustative aux concentrations délivrées sur les réseaux de distribution. L'odeur de chlore ajoutée à l'eau d'Evian a donc été utilisée pour l'application de la méthode à un problème concret de l'eau potable en France.

### Sujet

Pour rappel, l'ESPR utilisé ici correspond à un sujet ayant pour canal préférentiel la microcirculation cutanée. La représentation de l'ESPR de référence obtenue grâce à ses réponses physiologiques est donc déjà connue.

### Stimuli olfactif

Les concentrations ont donc été choisies en fonction des résultats obtenus sur les sujets français. Quatre concentrations différentes ont été utilisées (deux au niveau des seuils et deux plus évidentes) et sont décrites dans le tableau 5.

**Tableau 5 : Concentrations utilisées pour la saveur chlorée (en mg Cl₂.L⁻¹)**

| **Dilution** | **NaClO (Chlore)** |
|---|---|
| 1 | 0,10 |
| 2 | 0,17 |
| 3 | 0,32 |
| 4 | 1,00 |

Le chlore étant un produit hautement volatil, la solution mère a été préparée quelques minutes avant le début de la séance et les solutions ont été diluées dans les verres de dégustation une fois le sujet équipé et installé dans le box de dégustation.

### Matériel et méthode

Seul l'indicateur physiologique pertinent est mesuré, c'est-à-dire la microcirculation. Les paramètres à extraire et la standardisation des données sont déjà connus ce qui induit un gain de temps aux niveaux du traitement de l'information.

### Résultats

Les mesures déclarées sont présentées dans le tableau 6.

**Tableau 6 : Note d'intensité de chlore et hédonique déclarées**

| | Intensité chlorée | Note hédonique |
|---|---|---|
| Chlore 1 | 3,50 | 4,50 |
| Chlore 2 | 4,25 | 3,25 |
| Chlore 3 | 4,50 | 3,00 |
| Chlore 4 | 4,25 | 3,50 |

### Projection des nouveaux échantillons sur l'ESPR : réponse électrodermale :

La figure 7 présente la projection des valeurs des paramètres physiologiques mesurés pour les échantillons de Chlore. Les échantillons de chlore sont tous situés dans la partie droite du plan factoriel. Etant donné leurs coordonnées sur le premier facteur, il semble que l'ensemble des concentrations de chlore, même les plus faibles, a induit des réactions physiologiques fortes. L'étude des corrélations entre les deux premiers facteurs de l'ACP et les caractéristiques des échantillons chlorés confirment cette interprétation. En effet, la corrélation entre la note hédonique des stimuli chlorés et les deux premiers facteurs est significative et négative (respectivement r = -0,922, p < 0,10 et r = -0,889, p < 0,20). Il existe des corrélations positives entre l'intensité déclarée, la concentration en chlore et les deux premiers facteurs, mais qui n'atteignent pas le seuil de significativité de 20 %. La corrélation est positive entre le premier axe et l'intensité déclarée (r = 0,777, p = 0,22) et entre le deuxième facteur et la concentration (r = 0,662, p = 0,34). Il semble que le deuxième axe prenne une plus grande importance pour des échantillons chlorés et qu'il existe une relation d'ordre suivant F1 et F2. Ces résultats montrent que l'odeur de chlore induit des réponses bien plus fortes que les goûts de base.

**Exemple 5** : Utilisation des ESRP pour la prédiction des concentrations d'un produit Des modèles ont été construits, pour les saveurs acide, amère et salée, afin d'obtenir la prédiction des caractéristiques chimiques des stimuli dans l'eau. L'objectif est de mettre en évidence un lien direct entre les réactions physiologiques et la composition chimique des échantillons d'eau. La qualité de chaque modèle obtenu est présentée dans le tableau 7.

**Tableau 7 : Qualité des modèles pour la prédiction de la concentration des échantillons par type de goût, modèle de type Partial least square.**

| **Type de goût** | **Q² cum** |
|---|---|
| **Acide** | 0,980 |
| **Amer** | 0,920 |
| **Salé** | 0,995 |

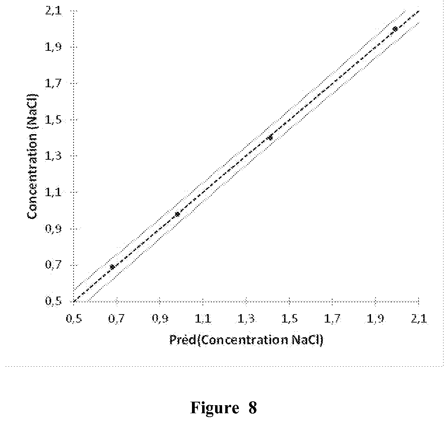

La figure 8 illustre la qualité des modèles de prédiction pour la saveur salée en comparant les concentrations réelles des échantillons de référence et leurs concentrations prédites.

Ces différents modèles ont été utilisés pour prédire les concentrations de solution référence. Le tableau suivant présente les concentrations réelles et prédites associées aux modèles PLS basé sur des paramètres physiologiques seuls. On remarque qu'il y a une bonne adéquation entre les différentes concentrations.

**Tableau 8 : Comparaison des concentrations réelles et prédites d'après chacun des modèles issus de la régression PLS sur les échantillons par type de goût**

| | Concentration (en mmol.L⁻¹ (g.L⁻¹)) | |
|---|---|---|
| | réelle | prédite |
| Acide 4_Exp2 | 3,12 (0,60) | 3,38 ± 0,05 (0,65 ± 0,01) |
| Caféine 3_Exp2 | 1,13 (0,22) | 1,28 ± 0,15 (0,25 ± 0,03) |
| NaCl 1_Exp2 | 11,81 (0,69) | 14,89 ± 0,34 (0,87 ± 0,02) |

## Revendications

1. Méthode de construction d'un espace sensoriel spécifique à un individu comprenant :
- la réalisation d'une évaluation sensorielle d'au moins 4 stimuli de base représentatifs du sens à étudier par l'individu, les stimuli ayant une intensité, inférieure à 3 fois l'intensité détectable par le sens à étudier;
- l'enregistrement des signaux relatifs à plusieurs indicateurs physiologiques ayant réagi lors des stimuli;
- l'extraction de paramètres physiologiques à partir des signaux enregistrés desdits indicateurs physiologiques;
- l'identification des paramètres physiologiques discriminants des réponses physiologiques par traitement statistique;
- la construction d'un espace sensoriel basé sur les réponses physiologiques (ESRP) à partir de paramètres physiologiques discriminants identifiés par traitement statistique.

2. Méthode selon la revendication 1, dans laquelle les indicateurs physiologiques sont choisis parmi les indicateurs du système nerveux central et/ ou du système nerveux autonome.

3. Méthode selon la revendication 2, dans laquelle le ou les indicateur(s) physiologique(s) du système nerveux central est (sont) l'activité du système nerveux central qui peut être mesurée par électroencéphalographie (EEG) ou par spectroscopie proche infrarouge fonctionnelle.

4. Méthode selon la revendication 2, dans laquelle le ou les indicateur(s) physiologique(s) du système nerveux autonome est (sont) choisis parmi la fréquence cardiaque, la réponse électrodermale, la microcirculation cutanée, le réflexe gusto-facial mesuré par électromyographie, la réponse pupillaire, la réponse respiratoire, la température cutanée et la pression sanguine.

5. Méthode selon l'une quelconque des revendications 1 à 4, dans laquelle l'identification des paramètres physiologiques discriminants est faite par au moins une analyse statistique choisie parmi l'analyse de la variance (ANOVA), le coefficient de Pearson, l'analyse en composantes principales (ACP), l'analyse factorielle des correspondances (AFC) ou des combinaisons de ces différentes méthodes.

6. Méthode d'analyse sensorielle d'un échantillon comprenant :
- la construction d'un espace sensoriel d'un individu telle que définie à l'une quelconque des revendications 1 à 5 ;
- l'évaluation de l'échantillon par l'individu ;
- l'enregistrement des signaux relatifs à au moins un indicateur physiologique correspondant aux paramètres physiologiques discriminants utilisés pour la construction de l'espace sensoriel ;
- l'extraction des paramètres physiologiques discriminants de l'échantillon ;
- le positionnement de l'échantillon dans l'espace sensoriel à partir des paramètres physiologiques discriminants de l'échantillon.

7. Méthode d'analyse selon la revendication 6, **caractérisée par le fait que** l'échantillon à analyser est un échantillon d'eau potable.

## Patentansprüche

1. Verfahren zur Konstruktion eines für ein Individuum spezifischen sensorischen Raumes, umfassend:
- Durchführung einer sensorischen Bewertung von mindestens vier Grundreizen, die für den von der Person zu untersuchenden Sinn repräsentativ sind, wobei die Reize eine Intensität haben, die weniger als das Dreifache der Intensität beträgt, die von dem zu untersuchenden Sinn erfasst werden kann,
- Aufzeichnung von Signalen, die sich auf eine Vielzahl von physiologischen Indikatoren beziehen, die auf die Reize reagiert haben,
- Extraktion von physiologischen Parametern aus den aufgezeichneten Signalen der physiologischen Indikatoren,
- Identifizierung der unterscheidenden physiologischen Parameter der physiologischen Reaktionen durch statistische Verarbeitung,
- Konstruktion eines auf der physiologischen Reaktion basierenden sensorischen Raums (ESRP) aus den durch statistische Verarbeitung identifizierten unterscheidenden physiologischen Parametern.

2. Verfahren nach Anspruch 1, wobei die physiologischen Indikatoren aus Indikatoren des Zentralnervensystems und/oder des autonomen Nervensystems gewählt werden.

3. Verfahren nach Anspruch 2, wobei der (die) physiologische(n) Indikator(en) des Zentralnervensystems die Aktivität des Zentralnervensystems ist (sind), die durch Elektroenzephalographie (EEG) oder funktionelle Nahinfrarotspektroskopie gemessen werden kann.

4. Verfahren nach Anspruch 2, wobei der (die) physiologische(n) Indikator(en) des autonomen Nervensystems gewählt wird (werden) aus Herzfrequenz, elektrodermaler Reaktion, Hautmikrozirkulation, gustofazialem Reflex gemessen durch Elektromyographie, Pupillenreaktion, Atemreaktion, Hauttemperatur und Blutdruck.

5. Verfahren nach einem der Ansprüche 1 bis 4, wobei die Identifizierung unterscheidender physiologischer Parameter durch mindestens eine statistische Analyse durchgeführt wird, die aus der Varianzanalyse (ANOVA), dem Pearson-Koeffizienten, der Hauptkomponentenanalyse (PCA), der Korrespondenzfaktoranalyse (CFA) oder Kombinationen davon gewählt wird.

6. Verfahren zur sensorischen Analyse einer Probe, umfassend:
- Konstruieren eines sensorischen Raums eines Individuums, wie in einem der Ansprüche 1 bis 5 definiert,
- Auswerten der Probe durch das Individuum,
- Aufzeichnen von Signalen, die sich auf mindestens einen physiologischen Indikator beziehen, der den unterscheidenden physiologischen Parametern entspricht, die zum Konstruieren des sensorischen Raums verwendet werden,
- Extrahieren der unterscheidenden physiologischen Parameter aus der Probe,
- Positionieren der Probe in dem sensorischen Raum auf der Basis der unterscheidenden physiologischen Parameter der Probe.

7. Analyseverfahren nach Anspruch 6, **dadurch gekennzeichnet, dass** die zu analysierende Probe eine Trinkwasserprobe ist.

## Claims

1. A method for constructing a sensory space specific to an individual, comprising:
- conducting a sensory evaluation of at least 4 basic stimuli representative of the sense to be studied by the individual, the stimuli having an intensity lower than 3 times the intensity detectable by the sense to be studied;
- recording signals relating to several physiological indicators which reacted during the stimuli;
- extracting physiological parameters from the signals recorded of said physiological indicators;
- identifying discriminating physiological parameters from the physiological responses by statistical analysis;
- constructing a sensory space based on the physiological responses (ESRP) from discriminating physiological parameters identified by statistical treatment.

2. The method as claimed in claim 1, wherein the physiological indicators are chosen from indicators of the central nervous system and/or the autonomic nervous system.

3. The method as claimed in claim 2, wherein the physiological indicator(s) of the central nervous system is (are) the activity of the central nervous system, which may be measured by electroencephalography (EEG) or by functional near-infrared spectroscopy.

4. The method as claimed in claim 2, wherein the physiological indicator(s) of the autonomic nervous system is (are) chosen from heart rate, electrodermal response, skin microcirculation, gustofacial reflex measured by electromyography, pupil response, respiratory response, skin temperature and blood pressure.

5. The method as claimed in any one of claims 1 to 4, wherein the discriminating physiological factors are identified by a statistical analysis chosen from analysis of variance (ANOVA), Pearson coefficient, principal component analysis (PCA), factorial correspondence analysis (FCA), or combinations of these different methods.

6. A method for the sensory analysis of a sample, comprising:
- constructing a sensory space of an individual as defined in any one of claims 1 to 5;
- evaluation of the sample by the individual;
- recording the signals relating to at least one physiological indicator corresponding to the discriminating physiological parameters used for constructing the sensory space;
- extracting the discriminating physiological parameters of the sample;
- positioning the sample in the sensory space using the discriminating physiological parameters of the sample.

7. The method of analysis as claimed in claim 6, **characterized in that** the sample to be analyzed is a sample of drinking water.
